# EUROPÄISCHE PATENTSCHRIFT

(11) **EP 2 033 622 B1**
(45) Veröffentlichungstag und Bekanntmachung des Hinweises auf die Patenterteilung: **06.03.2013**
(21) Anmeldenummer: 08014169.0
(22) Anmeldetag: 08.08.2008
(51) Int. Cl.: A61K 8/34, A61K 8/365, A61Q 19/02, A61Q 19/08, A61Q 19/10

(54) **Kosmetisches Reinigungsmittel mit niedrigem pH**
Cosmetic cleaning products with a low pH
Produit de nettoyage cosmétique ayant un pH réduit

(30) Priorität: 31.08.2007 DE 102007041494
(43) Veröffentlichungstag der Anmeldung: 11.03.2009
(73) Patentinhaber: Henkel AG & Co. KGaA, 40589 Düsseldorf (DE)
(72) Erfinder: Kreisig, Annette, 6291 CJ Vaals (NL); Schelges, Heike, 47877 Willich (DE)

(56) Entgegenhaltungen:
- DE-A1- 10 217 628
- DE-A1- 10 341 179
- DE-A1- 10 354 213
- DE-A1- 19 649 287
- DE-U1- 9 210 516
- US-A1- 2007 190 005
- US-B1- 6 486 106

## Beschreibung

Gegenstand der vorliegenden Anmeldung sind Reinigungsmittel für die Haut und/oder die Haare, die einen höheren Gehalt an alpha-Hydroxycarbonsäuren, alpha-Ketocarbonsäuren und/oder beta-Hydroxycarbonsäuren und damit einen relativ niedrigen pH-Wert aufweisen und die insbesondere zur Hautaufhellung, zur Aufhellung von Altersflecken, zur Egalisierung der Gesichtsfarbe, zur Verringerung der Tiefe von Hautfalten und/oder zur Egalisierung des Hautreliefs, verwendet werden können.

Das Patent US 6,486,106 offenbart Reinigungszusammensetzungen mit mildem Tensid und alpha-Hydroxycarbonsäuren. Ein zusätzlicher Gehalt an 1,6 Hexandiol wird darin nicht beschrieben.

DE 196 49 287 beschreibt tensidhaltige Reinigungszusammensetzungen mit einem Gehalt an 1,6 Hexandiol, allerdings ohne einen Gehalt an alpha- und/oder beta-Hydroxycarbonsäuren und/oder alpha-Ketocarbonsäuren zu offenbaren.

### Aufgabenstellung

Bei der Formulierung eines wasserbasierten Reinigungsmittels für die Haut und/oder die Haare, das einen höheren Gehalt an alpha-Hydroxycarbonsäuren, alpha-Ketocarbonsäuren und/oder beta-Hydroxycarbonsäuren und damit einen relativ niedrigen pH-Wert aufweist, muss auf jeden Fall ein ausreichendes Konservierungssystem gefunden werden, um die mikrobielle Stabilität des kosmetischen Produktes über einen längeren Zeitraum der Lagerung zu gewährleiten. Dies ist insbesondere wichtig, da die verwendeten alpha-Hydroxycarbonsäuren, alpha-Ketocarbonsäuren und/oder beta-Hydroxycarbonsäuren von vielen Mikroorganismen verstoffwechselt werden können, also als Nährstoff für Mikroorganismen dienen können. Überraschend wurde festgestellt, dass die Verwendung der üblichen Konservierungsmittel zusammen mit den verwendeten alpha-Hydroxycarbonsäuren, alpha-Ketocarbonsäuren und/oder beta-Hydroxycarbonsäuren diverse Nachteile aufwarf: eine Benzoesäure-basierte bzw. Benzoat-basierte Konservierung führte zu starken Hautunverträglichkeitsreaktionen. Bei Zusatz von Parabenen und/oder Phenoxyethanol bzw. Phenoxypropanol traten unerwünschte Trübungen und Farbveränderungen des Produktes auf. Mit anderen üblichen Konservierungsstoffen in den zugelassenen Höchstmengen konnte keine befriedigende mikrobielle Stabilität erzielt werden.

Dementsprechend lag der vorliegenden Anmeldung die Aufgabe zugrunde, ein wasserbasiertes Reinigungsmittel mit einem höheren Gehalt an alpha-Hydroxycarbonsäuren, alpha-Ketocarbonsäuren und/oder beta-Hydroxycarbonsäuren und damit einen relativ niedrigen pH-Wert ausreichend lagerstabil zu konservieren und gleichzeitig ein möglichst gut hautverträgliches, auch von seinem äußeren Erscheinungsbild her ästhetisches Produkt zu formulieren.

Überraschend wurde festgestellt, dass die gestellten Aufgaben hervorragend gelöst werden können durch die Kombination eines milden Tensidsystems mit einem höheren Gehalt an alpha-Hydroxycarbonsäuren, alpha-Ketocarbonsäuren und/oder beta-Hydroxycarbonsäuren und einem Zusatz von mindestens 2 Gew.-% 1,6-Hexandiol.

Gegenstand der vorliegenden Anmeldung ist daher eine Reinigungszusammensetzung für die Haut und/oder die Haare, enthaltend Wasser, ein mildes Tensidsystem, umfassend 0,5 - 20 Gew.-% Gesamttensidgehalt, mindestens 2 Gew.-% mindestens einer alpha-Hydroxycarbonsäure, alpha-Ketocarbonsäure und/oder beta-Hydroxycarbonsäure, mindestens 2 Gew.-% 1,6-Hexandiol, wobei alle Mengenangaben auf das Gewicht der gesamten Zusammensetzung bezogen sind und wobei die Zusammensetzung frei ist von Cocamidopropylbetain, den Aniontensiden Seifen, Alkylbenzolsulfonaten, Alkansulfonaten, Olefinsulfonaten, Alkylethersulfonaten, Glycerinethersulfonaten, α -Methylestersulfonaten, Sulfofettsäuren, Alkylsulfaten, Fettalkoholethersulfaten, Fettsäureamid(ether)sulfaten, Sulfotriglyceriden, Amidseifen, und Alkyl(ether)phosphaten, Benzoesäure, 4-Hydroxybenzoesäure, den Salzen und den Estern dieser Säuren, Phenoxyethanol, Phenoxypropanol, lodopropynyl Butylcarbamate und 1,3-Bis(hydroxymethyl)-5,5-dimethylimidazolidin-2,4-dion, und wobei die gesamte Zusammensetzung einen pH-Wert im Bereich von 3,5 -4,5 aufweist.

Die Mengenangaben zu den alpha-Hydroxycarbonsäuren, alpha-Ketocarbonsäuren und beta-Hydroxycarbonsäuren schließen sowohl die freie Säure als auch die korrespondierende(n) Base(n) ein, also bei einbasigen Säuren, wie Glycolsäure, sowohl die freie Säure als auch das Glycolatanion, bei mehrbasigen Säuren, wie Citronensäure, sowohl die freie Säure als auch alle Dissoziationsformen.

Ein zentrales obligatorisches Merkmal der erfindungsgemäßen Reinigungszusammensetzungen ist ein Mindestgehalt an 1,6-Hexandiol von 2 Gew.-%. Bevorzugt ist ein Gehalt an 1,6-Hexandiol von 4 - 20 Gew.-%, besonders bevorzugt 6 - 10 Gew.-%, jeweils bezogen auf das Gewicht der gesamten Zusammensetzung. Überraschend wurde festgestellt, dass 1,6-Hexandiol in den beanspruchten Mengen eine sehr zufrieden stellende mikrobielle Stabilität der erfindungsgemäßen Zusammensetzungen ermöglicht, und zwar auch in Abwesenheit der üblicherweise verwendeten Konservierungsmittel Benzoesäure, 4-Hydroxybenzoesäure, den Salzen und den Estern der Benzoesäure und der 4-Hydroxybenzoesäure, Phenoxyethanol, Phenoxypropanol, lodopropynyl Butylcarbamate und 1,3-Bis(hydroxymethyl)-5,5-dimethylimidazolidin-2,4-dion. Gleichzeitig zeigen die erfindungsgemäßen Zusammensetzungen eine hervorragende Hautverträglichkeit. Außerdem ist die Formulierung transluzenter und sogar transparenter Zusammensetzungen möglich.

Ein weiteres obligatorisches Merkmal der erfindungsgemäßen Reinigungszusammensetzungen ist ein mildes Tensidsystem mit einem Gesamttensidgehalt von 0,5 - 20 Gew.-%, bevorzugt 1 - 10 Gew.-%, besonders bevorzugt 2 - 6 Gew.-%, jeweils bezogen auf das Gewicht der gesamten erfindungsgemäßen Zusammensetzung, und das frei ist von Cocamidopropylbetain und den Aniontensiden Seifen, Alkylbenzolsulfonaten, Alkansulfonaten, Olefinsulfonaten, Alkylethersulfonaten, Glycerinethersulfonaten, a-Methylestersulfonaten, Sulfofettsäuren, Alkylsulfaten, Fettalkoholethersulfaten, Fettsäureamid(ether)sulfaten, Sulfotriglyceriden, Amidseifen und Alkyl(ether)phosphaten. Außerordentlich bevorzugt ist ein Gesamttensidgehalt von maximal 4,5 Gew.-%, bezogen auf die gesamte Zusammensetzung.

Unter dem Begriff "milde Tenside" versteht der Fachmann Tenside, die sich in den zahlreichen Testmethoden, wie dem HET-CAM Test, dem Neutralrottest, dem BUS - Modell (bovine udder skin model), dem Humanhautmodell, dem Zeintest, dem Draize Test, dem Armflexwashtest oder dem Duhringkammertest usw. als milde Tenside erwiesen haben. Allen Testmodellen gemein ist, dass prinzipiell gegen einen Standard gemessen wird, auf den die Messergebnisse bezogen werden. In jedem dieser Testmodelle gibt es einen Schwellenwert, unterhalb dessen von "milden Tensiden" gesprochen wird. Dieser Schwellenwert beträgt beispielsweise im HET-CAM-Test 1,5. Das bedeutet, dass als "mild" alle Tenside bezeichnet werden, die beispielsweise im HET-CAM-Test einen relativen Reizscore von 1,5 und kleiner aufweisen. Der Fachmann weiß, dass ein Tensid in jedem Testmodell einen anderen Score ergibt. Das bedeutet, dass beispielsweise ein Cocamidopropylbetain im HET-CAM-Test sogar als "reizend" eingestuft sein kann, während es in den anderen Testmodellen eher den milden Tensiden zugerechnet wird. Eine geläufige und anerkannte Einstufung definiert Tenside als mild, wenn sie im HET-CAM-Test einen relativen Reizscore von kleiner als 1,5 aufweisen. Erfindungsgemäß bevorzugt werden jedoch solche Tenside als "milde Tenside" verwendet und verstanden, die in allen derzeit geläufigen Testmodellen als "mild" eingestuft werden. Besonders bevorzugt werden als milde Tenside solche Tenside verwendet, die im HET-CAM-Test einen relativen Reizscore von kleiner 1,2 aufweisen. Ganz besonders bevorzugt werden als milde Tenside solche Tenside verwendet, die einen relativen Reizscore im HET-CAM-Test von kleiner 0,8 aufweisen. In allen Fällen werden die entsprechenden HET-CAM-Tests mit einer Tensidkonzentration von 3,0 Gew.-% Aktivsubstanz des jeweiligen Tensids in einer wässrigen Lösung durchgeführt.

Bevorzugte erfindungsgemäße Reinigungszusammensetzungen enthalten mindestens ein C₆-C₁₆-Alkyl- oder Alkenylglycosid mit einem Oligomerisierungsgrad von 1 - 3. Bevorzugte C₆-C₁₆-Alkyl- oder Alkenylglycosid mit einem Oligomerisierungsgrad von 1 - 3 weisen die allgemeine Formel R^{APG}O-[G]ₚ auf, in der R^{APG} für einen Alkyl- oder Alkenylrest mit 4 bis 22 Kohlenstoffatomen, G für einen Zuckerrest mit 5 oder 6 Kohlenstoffatomen und p, der Oligomerisierungsgrad, für Zahlen von 1 bis 10 steht. Diese Verbindungen können nach den einschlägigen Verfahren der präparativen organischen Chemie erhalten werden. Die Alkyl- und Alkenyloligoglycoside können sich von Aldosen bzw. Ketosen mit 4, 5 oder 6 Kohlenstoffatomen ableiten. Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die bevorzugten Alkyl- und/oder Alkenyloligoglycoside sind somit Alkyl- und/oder Alkenyloligoglucoside. Die Indexzahl p gibt den Oligomerisierungsgrad (DP), d. h. die Verteilung von Mono- und Oligoglycosiden, an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alk(en)yloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl-und/oder Alkenyloligoglycoside bevorzugt, deren Oligomerisierungsgrad kleiner als 1,5 ist und insbesondere 1,1 bis 1,4 beträgt. Der Alkyl- bzw. Alkenylrest R^{APG} kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3, bevorzugt 1,2 - 1,4), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3, bevorzugt 1,2 - 1,4). Der Alkyl- bzw. Alkenylrest R^{APG} kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3, bevorzugt 1,1-1,4.

Bevorzugte erfindungsgemäße Zusammensetzungen enthalten die im Folgenden beschriebenen milden anionischen Tenside.
Nach den oben genannten Prüfmethoden haben sich die folgenden anionischen Tenside als mild bis besonders mild erwiesen und sind erfindungsgemäß besonders bevorzugt:
- Acyllactylate,
- Hydroxymischethersulfate,
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist und deren Salze,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, erhältlich durch Veresterung von Fettsäuren mit dem Natriumsalz der 2-Hydroxyethansulfonsäure (Isethionsäure). Wenn man für diese Veresterung Fettsäuren mit 8 bis 24 C-Atomen, also z. B. Laurin-, Myristin-, Palmitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate;
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen. Die Sulfobernsteinsäuremonoalkyl(C₈-C₂₄)-ester-dinatriumsalze werden nach bekanntem Verfahren z. B. dadurch hergestellt, dass man Maleinsäureanhydrid mit einem Fettalkohol mit 8 - 24 C-Atomen zum Maleinsäuremonoester des Fettalkohols umsetzt und diesen mit Natriumsulfit zum Sulfobernsteinsäureester sulfitiert. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind.
- Ester der Weinsäure und Zitronensäure mit Alkoholen, die Anlagerungsprodukte von 2 - 15 Molekülen Ethylenoxid und/oder Propylenoxid an Fettalkohole mit 8 bis 22 C-Atomen darstellen,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für ein Wasserstoffion oder ein Metallion wie ein Alkalimetallion, insbesondere die Kationen von Natrium, Kalium, Lithium, weiterhin ein Erdalkalimetallion, insbesondere die Kationen von Magnesium, Calcium, Zink, oder ein Ammoniumion, wie ⁺NR³R⁴R⁵R⁶, mit R³ bis R⁶ unabhängig voneinander stehend für Wasserstoff oder einen C₁-C₄ - Kohlenwasserstoffrest. Derartige Produkte sind beispielsweise von der Firma Chem-Y unter der Produktbezeichnung Akypo^{®}erhältlich.
- Kondensationsprodukte aus einem wasserlöslichen Salz eines wasserlöslichen Eiweißhydrolysat-Fettsäure-Kondensationsprodukts. Diese werden durch Kondensation von C₈ - C₃₀ Fettsäuren, bevorzugt von Fettsäuren mit 12 - 18 C-Atomen mit Aminosäuren, Mono-, Di- und wasserlöslichen Oligopeptiden und Gemischen solcher Produkte hergestellt, wie sie bei der Hydrolyse von Proteinen anfallen. Diese Eiweißhydrolysat-Fettsäure-Kondensationsprodukte werden mit einer Base neutralisiert und liegen dann bevorzugt als Alkali-, Ammonium-, Mono-, Di- oder Trialkanolammoniumsalz vor. Solche Produkte sind unter dem Warenzeichen Lamepon^{®}, Maypon^{®}, Gluadin^{®}, Hostapon^{®} KCG oder Amisoft^{®} seit langem im Handel erhältlich,
- Acylglutamate und
- Acylaspartate.

Sofern die milden anionischen Tenside Polyglycoletherketten (Polyethylenoxid-Ketten) enthalten, ist es ganz besonders bevorzugt, dass diese eine eingeengte "narrow range" Homologenverteilung aufweisen. Weiterhin ist es im Falle von milden anionischen Tensiden mit Polyglycolethereinheiten bevorzugt, dass die Zahl der Glycolethergruppen (= Ethylenoxideinheiten) 1 bis 20 beträgt, bevorzugt 2 bis 15, besonders bevorzugt 2 bis 12. Besonders milde anionische Tenside mit Polyglycolethergruppen ohne eingeschränkte Homologenverteilung können beispielsweise auch erhalten werden, wenn einerseits die Zahl der Polyglycolethergruppen (= Ethylenoxideinheiten) 4 bis 12 beträgt und als Gegenion Zn-oder Mg-lonen gewählt werden.

Selbstverständlich können alle bislang und im Folgenden genannten milden anionischen Tenside auch in Form ihrer Salze verwendet werden. Besonders geeignete milde anionische Tenside liegen jeweils in Form der Lithium-, Magnesium-, Zink-, Natrium-, Kalium- und Ammonium- sowie der Mono-, Di- und Trialkanolammoniumsalze mit 1 bis 4 C-Atomen in der Alkanolgruppe vor. Die bevorzugten Ammoniumionen sind neben dem Ammoniumion als solchem Monomethylammonium-, Dimethylammonium-, Trimethylammonium-, Monoethylammonium-, Diethylammonium-, Triethylammonium-, Monopropylammonium-, Dipropylammonium-, Tripropylammonium-, Monoisopropylammonium-, Diisopropylammonium-, Triisopropylammonium-, Monobutylammonium-, Dibutylammonium-, Tributylammonium-, Monoisobutylammonium-, Diisobutylammonium-, Triisobutylammonium-, Mono-t-butyl-ammonium-, Dit-butyl-ammonium-, Tri-t-butyl-ammoniumionen sowie gemischte Ammoniumionen wie beispielsweise Methyl-ethyl-ammonium-, Dimethyl-ethyl-ammonium-, Methyl-diethylammonium-, Methyl-propyl-ammonium-, Methyl-ethyl-propyl-ammonium-, Ethyl-diisopropylammonium-, Ethyl-dibutyl-ammonium-, Ethyl-diisobutylammoniumionen usw. Selbstverständlich umfasst die erfindungsgemäße Lehre auch die weiteren nicht explizit genannten Ammoniumionen dieser Alkanolammoniumsalze.

Weitere milde anionische Tenside, die ganz besonders bevorzugt in der erfindungsgemäßen Zusammensetzung verwendet werden, sind Alkyl- und/oder Alkenyl-Oligoglycosidcarboxylate, -sulfate, -phosphate und/oder -isethionate, die sich von Alkyl- und/oder Alkenyloligoglycosiden der allgemeinen Formel R-O-(G)_{P} ableiten, mit der Bedeutung
- R: C₆₋₂₂-Alkyl oder C₆₋₂₂-Alkenyl,
- G: Glycosideinheit, die sich von einem Zucker mit 5 oder 6 Kohlenstoffatomen ableitet,
- p: Zahl von 1 bis 10.

Bevorzugt ist das milde anionische Tensid ausgewählt aus den anionischen Alkylpolyglycosiden, den Ethercarbonsäuren, den Acylisethionaten, den Eiweißfettsäurekondensaten, den Tauraten, den Sulfosuccinaten, den Fettsäureamidethersulfaten, den Acylglutamaten, den Acylasparaginaten und deren Mischungen.
Erfindungsgemäß werden besonders bevorzugt die anionischen Alkylpolyglucoside, wie Alkyloligoglycosidcarboxylate, -sulfate, -phosphate und/oder -isethionate, Ethercarbonsäuren, Acylisethionate sowie Taurate und deren Mischungen eingesetzt.
Ganz besonders bevorzugt ist die Verwendung von anionischen Alkylpolyglucosiden und Ethercarbonsäuren sowie deren Mischungen.
Höchst bevorzugt ist die Verwendung von carboxylierten Alkylpolyglucosiden und Ethercarbonsäuren sowie deren Mischungen.
Werden als mildes anionisches Tensid Mischungen aus zwei unterschiedlichen milden anionischen Tensiden verwendet, so beträgt das Gewichtsverhältnis dieser Tenside zueinander 10:1 bis 1 :10. Bevorzugt ist ein Gewichtsverhältnis von 5 : 1 bis 1 : 5, besonders bevorzugt von 2,5 : 1 bis 1 : 2,5 und am bevorzugtesten von etwa 1,5 :1 bis 1 : 1,5.

Es wurde erfindungsgemäß gefunden, dass die Verwendung von milden anionischen Tensiden und insbesondere von Alkyl- und/oder Alkenyl-Oligoglycosidcarboxylaten, -sulfaten, -phosphaten und/oder -isethionaten in den erfindungsgemäßen Mitteln zu einer Verminderung der Hautreizung führt.
Darüber hinaus wurde erfindungsgemäß gefunden, dass bei Verwendung milder anionischer Tenside, insbesondere der Alkyl- und/oder Alkenyl-Oligoglycosidcarboxylate, -sulfate, -phospate und/oder -isethionate in Mitteln zur Reinigung und Pflege bei der Anwendung dieser Mittel das Aufschäumen erheblich verbessert wird. Der Schaum zeichnet sich insbesondere durch ein feinporiges, dichtes, cremiges Erscheinungsbild aus. Der Schaum wird als angenehm weich und geschmeidig und leicht verteilbar beschrieben. Gleichzeitig ist der Schaum fest und gut greifbar. Er zeigt ein gewisses Standvermögen und verläuft nicht spontan, sondern erst nach einigen Minuten. Dies begünstigt die bereits beschriebene leichte Verteilbarkeit des Schaums. Diese Effekte treten in den erfindungsgemäßen Zusammensetzungen insbesondere dann auf, wenn weiterhin in Kombination mit kationischen und / oder amphoteren Polymeren formuliert wird.
Für die Alkyl- und/oder Alkenyl-Oligoglycosidcarboxylate, -sulfate, -phosphate und -isethionate gilt für die Glycosid-Einheiten G und den Oligomerisierungsgrad p das vorstehend zu den Alkyl- und Alkenyl-Oligoglycosiden der allgemeinen Formel R^{APG}O-[G]ₚ Gesagte.
In den Alkyl- und/oder Alkenyl-Oligoglycosiden ist vorzugsweise in mindestens einem der Reste G mindestens eine Hydroxylgruppe durch -O-C₁₋₁₂-Alkenyl-COOM, -OSO₃M, -OP(O)(OM)₂ oder -O-CH₂-CH₂-SO₃M mit M = H, Alkalimetall, NH₄ oder einem der bereits zuvor genannten Gegenionen wie Zn, Mg, Alkanolammonium ersetzt.
Dabei wird besonders bevorzugt ein Alkyloligoglycosid-Carboxylat eingesetzt, in dem -O-C₁₋₁₂-Alkylen-COOM -O(CH₂-)ₙCOOM mit M = H, Na oder K und n = 1 bis 3 bedeutet, das heißt, bevorzugte Alkylengruppen sind die Methylengruppe, die Ethylengruppe und die Propylengruppe. Besonders bevorzugt ist der Rest O-CH₂-COONa.
Besonders bevorzugt wird ein Alkyloligoglycosidcarboxylat eingesetzt, in dem der Alkylrest ein Laurylrest ist. Speziell bevorzugt ist ein Laurylglucosidcarboxylat, wie es als Plantapon^{®} LCG von Cognis Deutschland erhältlich ist.
Der Alkylrest R leitet sich von primären Alkoholen mit 6 bis 22, vorzugsweise 12 bis 18 Kohlenstoffatomen ab. Typische Beispiele sind Capronalkohol, Caprylalkohol, Caprinalkohol, Laurylalkohol, Myristylalkohol, Cetylalkohol, Stearylalkohol und Behenylakohol sowie technische Fraktionen, die neben den genannten gesättigten Alkoholen auch Anteile an ungesättigten Alkoholen enthalten können und die auf Basis von natürlichen Fetten und Ölen, beispielsweise Palmöl, Palmkernöl, Kokosöl oder Rindertalg gewonnen werden. Der Einsatz von technischem Kokosalkohol ist hierbei besonders bevorzugt.
Neben den genannten Fettalkoholen können sich die Alkylglycoside auch von synthetischen primären Alkoholen mit 6 bis 22 Kohlenstoffatomen, insbesondere den sogenannten Oxoalkoholen ableiten, die einen Anteil von 5 bis 40 Gew.-% verzweigter Isomeren aufweisen.
Besonders bevorzugte Alkylreste sind solche mit 8/10, 12/14, 8 bis 16, 12 bis 16 oder 16 bis 18 C-Atomen. Mischungen der Alkylreste ergeben sich bei einer Herstellung ausgehend von natürlichen Fetten und Ölen oder Mineralölen.

Weitere erfindungsgemäß bevorzugte Reinigungszusammensetzungen sind dadurch gekennzeichnet, dass mindestens ein kationisches Tensid, ausgewählt aus der Tensidklasse der Esterquats, enthalten ist.
Unter der Bezeichnung Esterquats werden im Allgemeinen quaternierte Fettsäuretriethanolaminestersalze verstanden. Es handelt sich dabei um bekannte Stoffe, die man nach den einschlägigen Methoden der präparativen organischen Chemie erhalten kann.
Bevorzugte quaternierte Fettsäuretriethanolaminestersalze weisen die allgemeine Formel (EQ-1) auf 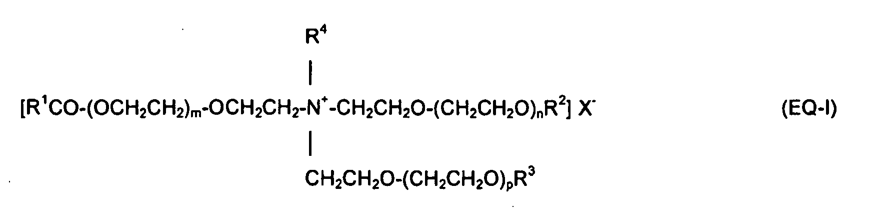 in der R¹CO für einen Acylrest mit 6 bis 22 Kohlenstoffatomen, R² und R³ unabhängig voneinander für Wasserstoff oder R¹CO, R⁴ für einen Alkylrest und/oder Hydroxyalkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X- für Halogenid, Alkylsulfat, insbesondere Methylsulfat, oder Alkylphosphat steht.
Typische Beispiele für erfindungsgemäß bevorzugte Esterquats sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg-bzw. Palmfettsäuren sowie Elaidinsäure-reiche C_{16/18}-Fettsäureschnitte eingesetzt.
Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab.
Aus anwendungstechnischer Sicht haben sich quaternierte Fettsäuretriethanolaminestersalze der Formel (EO-I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.
Neben den quaternierten Fettsäuretriethanolaminestersalzen kommen als bevorzugte Esterquats ferner auch quaternierte Estersalze von Fettsäuren mit Diethanolalkylaminen der Formel (EQ-II) in Betracht, 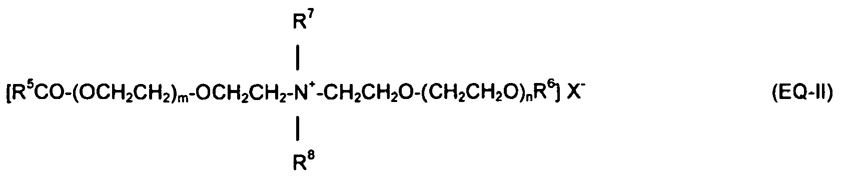 in der R⁵CO für einen Acylrest mit 6 bis 22 Kohlenstoffatomen, R⁶ für Wasserstoff oder R⁵CO, R⁷ und R⁸ unabhängig voneinander für Alkylreste und/oder Hydroxyalkylreste mit 1 bis 4 Kohlenstoffatomen, m und n in Summe für 0 oder Zahlen von 1 bis 12 und X- für Halogenid, Alkylsulfat, insbesondere Methylsulfat, oder Alkylphosphat steht.
Als weitere Gruppe bevorzugter Esterquats sind die quaternierten Estersalze von Fettsäuren mit 1,2-Dihydroxypropyldialkylaminen der Formel (EQ-III) zu nennen, 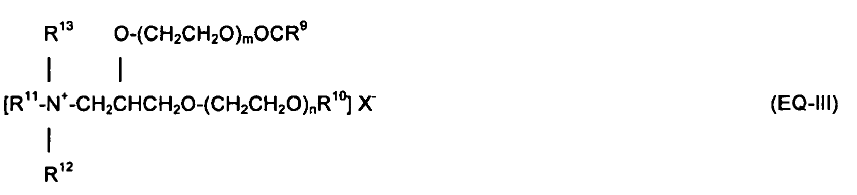 in der R⁹CO für einen Acylrest mit 6 bis 22 Kohlenstoffatomen, R¹⁰ für Wasserstoff oder R⁹CO, R¹¹, R¹² und R¹³ unabhängig voneinander für Alkylreste und/oder Hydroxyalkylreste mit 1 bis 4 Kohlenstoffatomen, m und n in Summe für 0 oder Zahlen von 1 bis 12 und X für Halogenid, Alkylsulfat, insbesondere Methylsulfat, oder Alkylphosphat steht.
Hinsichtlich der Auswahl der bevorzugten Fettsäuren und des optimalen Veresterungsgrades gelten die für (EQ-I) genannten Beispiele auch für die Esterquats der Formeln (EQ-II) und (EQ-III). Schließlich kommen als bevorzugte Esterquats noch Stoffe in Frage, bei denen die Esterbindung durch eine Amidbindung ersetzt ist und die, vorzugsweise basierend auf Diethylentriamin, der Formel (EQ-IV) folgen, 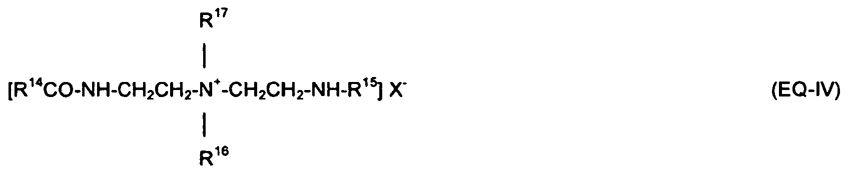 in der R¹⁴CO für einen Acylrest mit 6 bis 22 Kohlenstoffatomen, R¹⁵ für Wasserstoff oder R¹⁴CO, R¹⁶ und R¹⁷ unabhängig voneinander für Alkylreste mit 1 bis 4 Kohlenstoffatomen und Y für Halogenid, Alkylsulfat, insbesondere Methylsulfat, oder Alkylphosphat steht. Derartige Amidesterquats sind beispielsweise unter der Marke Incroquat® (Croda) im Markt erhältlich.

Eine weitere erfindungsgemäß bevorzugte Esterquat-Verbindung weist die INCI-Bezeichnung Quaternium-92 und die Formel (EQ-V) auf, 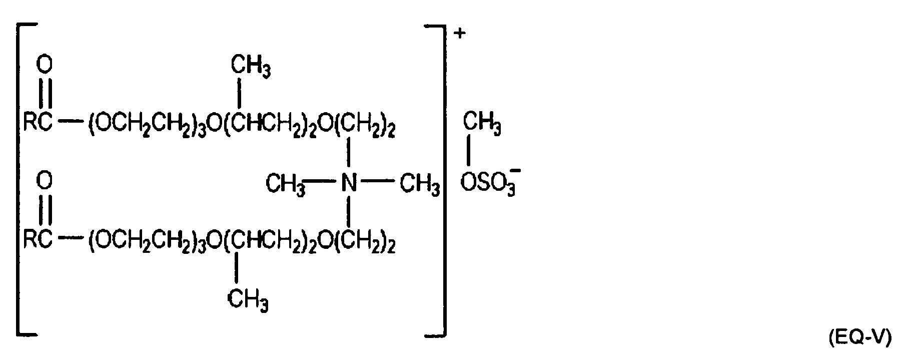 wobei die Reste RCO Lanolinsäure-Reste darstellen.

Erfindungsgemäß besonders bevorzugt sind Esterquats der Formel (EQ-II), in der R⁶ = R⁵CO gilt und R⁵CO einen Stearoylrest oder Cocoylrest (Octanoyl-/Decanoyl-/Lauroyl-/Myristoyl-/Cetoyl-/ Stearoyl-Gemisch), R⁷ eine -CH₂CH₂OH-Gruppe, R⁷ eine Methylgruppe und X⁻ eine Methylsulfat-Gruppe darstellt und m = n = 0 gilt. Außerordentlich bevorzugt sind Esterquats der Formel (EQ-II), in der R⁶ = R⁵CO gilt und R⁵CO einen Cocoylrest (Octanoyl-/Decanoyl-/Lauroyl-/Myristoyl-/Cetoyl-/Stearoyl-Gemisch), R⁷ eine -CH₂CH₂OH-Gruppe, R⁷ eine Methylgruppe und X⁻ eine Methylsulfat-Gruppe darstellt und m = n = 0 gilt.

Ein weiteres obligatorisches Merkmal der erfindungsgemäßen Reinigungszusammensetzungen ist ein Gehalt an mindestens 2 Gew.-%, bezogen auf das Gewicht der gesamten Zusammensetzung, mindestens einer alpha-Hydroxycarbonsäure, alpha-Ketocarbonsäure und/oder beta-Hydroxycarbonsäure. Da die erfindungsgemäßen Zusammensetzungen einen pH-Wert von 3,5 - 4,5 aufweisen, versteht es sich von selbst, dass - in Abhängigkeit von ihrem pKa-Wert - ein Teil der alpha-Hydroxycarbonsäuren, alpha-Ketocarbonsäuren und/oder beta-Hydroxycarbonsäuren in der Salzform vorliegt. Für die Bestimmung der Menge wird angenommen, dass die gesamte Säuremenge in der undissoziierten Säureform vorliegt.
Erfindungsgemäß bevorzugte alpha-Hydroxycarbonsäuren alpha-Ketocarbonsäuren oder beta-Hydroxycarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte alpha-Hydroxycarbonsäuren sind Glycolsäure und Gluconsäure. Eine besonders bevorzugte beta-Hydroxycarbonsäure ist Salicylsäure. Außerordentlich bevorzugt, insbesondere in Bezug auf die Hautverträglichkeit, ist Glycolsäure. Erfindungsgemäß besonders bevorzugte Zusammensetzungen sind dadurch gekennzeichnet, dass sie mindestens eine alpha-Hydroxycarbonsäure, alpha-Ketocarbonsäure und/oder beta-Hydroxycarbonsäure in einer Gesamtmenge von 2,5 - 5 Gew.-%, bevorzugt 3 - 4,5 Gew.-%, besonders bevorzugt 3,5 - 4 Gew.-%, jeweils bezogen auf die gesamte Zusammensetzung, enthalten.

In einer weiteren bevorzugten Ausführungsform enthält die erfindungsgemäße Reinigungszusammensetzung mindestens eine Guanidino-substituierte C₁ - C₁₀-Carbonsäure. Unter der Guanidino-Gruppe wird nach IUPAC-Regel C-961.2 und R-9.1.31 b die Gruppierung -NH-C(NH₂)=NH verstanden. Bevorzugt sind Guanidino-substituierte C₂ - C₁₀-alpha-Aminocarbonsäuren, insbesondere alpha-Aminoessigsäure, alpha-Aminopropansäure, alpha-Aminobutansäure, alpha-Aminopentansäure, alpha-Aminohexansäure, alpha-Aminoheptansäure, alpha-Aminooctansäure, alpha-Aminononansäure und alpha-Aminodecansäure. Bevorzugt tragen diese Säuren die Guanidino-Gruppe in der omega-Position, insbesondere 2-Amino-2-guanidinoessigsäure, 2-Amino-3-guanidinopropansäure, 2-Amino-4-guanidi-nobutansäure, 2-Amino-5-guanidinopentansäure, 2-Amino-6-guanidinohexansäure, 2-Amino-7-guani-dinoheptansäure, 2-Amino-8-guanidinooctansäure, 2-Amino-9-guanidinononansäure und 2-Amino-10-Guanidinodecansäure. Erfindungsgemäß außerordentlich bevorzugt ist 2-Amino-5-guanidinopentan-säure. Überraschend wurde festgestellt, dass der Zusatz einer Guanidino-substituierte C₁ - C₁₀-Carbonsäure, insbesondere einer alpha-Amino-omega-GuanidinO-C₂ - C₁₀-Carbonsäure, die Hautverträglichkeit weiter verbessert. Bevorzugt ist ein Gehalt an Guanidino-substituierte C₁ - C₁₀-Carbonsäure, bevorzugt an alpha-Amino-omega-Guanidino-C₂ - C₁₀-Carbonsäure, von 0,05 - 2 Gew.-%, besonders bevorzugt von 0,1 - 1 Gew.-%, außerordentlich bevorzugt von 0,2 - 0,6 Gew.-%, jeweils bezogen auf das Gewicht an freier Säure in der gesamten Zusammensetzung. Auch hier wird bei der Bestimmung des Mengenanteils von der Einwaage an freier Säure ausgegangen, unabhängig davon, dass in der Zusammensetzung selbst ein Teil der Säure als Salz vorliegt.

Weitere erfindungsgemäß bevorzugte Reinigungszusammensetzungen sind dadurch gekennzeichnet, dass das Gewichtsverhältnis von Gesamtmenge an alpha-Hydroxycarbonsäure, alpha-Ketocarbonsäure und beta-Hydroxycarbonsäure zur Gesamtmenge an Guanidino-substituierten C₁ - C₁₀-Carbonsäuren 4 - 20, bevorzugt 6 - 10, beträgt.

Die erfindungsgemäßen Reinigungszusammensetzungen enthalten Wasser. Bevorzugte erfindungsgemäße Reinigungszusammensetzungen enthalten 20 - 95 Gew.-% Wasser, besonders bevorzugt 40 - 90 Gew.-%, außerordentlich bevorzugt 60 - 85 Gew.-% Wasser, jeweils bezogen auf die gesamte Zusammensetzung. Auch Wassergehalte von 70 - 80 Gew.-% können bevorzugt sein.

Die erfindungsgemäßen Reinigungszusammensetzungen weisen einen pH-Wert im Bereich von 3,5 - 4,5, bevorzugt von 3,7 - 4,3, besonders bevorzugt von 3,8 - 4,2 und außerordentlich bevorzugt von 3,9-4-4,1, auf.

Die erfindungsgemäßen Reinigungszusammensetzungen müssen in einer anwenderfreundlichen, praktischen Darreichungsform konfektioniert werden.
In verdickter Form werden die erfindungsgemäßen Reinigungszusammensetzungen beispielsweise als Reinigungsgel, Reinigungsgelcreme oder Reinigungscreme angeboten. Zur Anwendung auf der Haut werden sie vom Verbraucher, beispielsweise mit der Hand, aber auch direkt aus dem Spender, auf die zu reinigende Haut appliziert. Auf diese Weise kann selbstverständlich auch das Haar behandelt werden.
In unverdickter Form, mit Wasser-ähnlicher Konsistenz, können die erfindungsgemäßen Reinigungszusammensetzungen beispielsweise als Gesichtswasser oder Gesichts-Tonic oder Hair-Tonic angeboten werden. Zur Anwendung auf der Haut werden sie zuvor vorteilhafterweise vom Verbraucher auf ein Substrat, beispielsweise ein Tuch, Schwamm, Pad oder einen (Watte-)Bausch aufgetragen und mit dem getränkten Substrat auf die Haut appliziert. Mit diesem getränkten Substrat kann selbstverständlich auch das Haar behandelt werden.

In einer bevorzugten Ausführungsform der Erfindung verbleibt die Reinigungszusammensetzung für eine gewisse Einwirkzeit, beispielsweise für 1 Sekunde bis 5 Minuten, auf der Haut und/oder dem Haar. Einwirkzeiten von 10 Sekunden bis 3 Minuten, bevorzugt 20 Sekunden bis 2 Minuten, besonders bevorzugt 30 Sekunden bis 1 Minute, sind ebenfalls erfindungsgemäß. Außerordentlich bevorzugt ist eine Einwirkzeit von 2 - 5 Minuten. Nach dieser Einwirkzeit kann die erfindungsgemäße Zusammensetzung abgespült, vorzugsweise mit Wasser, abgespült werden. Die erfindungsgemäße Zusammensetzung kann auch mit einem Substrat, beispielsweise mit einem Tuch, Waschlappen, Schwamm, Pad oder einem (Watte-) Bausch, abgewischt oder abgetupft oder in sonstiger Form entfernt werden. Bevorzugt ist das zum Abtragen verwendete Substrat, beispielsweise Tuch, Waschlappen, Schwamm, Pad oder (Watte-)Bausch, mit Wasser getränkt.

Ebenfalls erfindungsgemäß bevorzugt ist eine Substrat-freie Applikation der erfindungsgemäßen Zusammensetzung auf die Haut und/oder die Haare. Hierzu sollte die erfindungsgemäße Zusammensetzung verdickt sein. Eine lagerstabile Verdickung der erfindungsgemäßen Zusammensetzungen ist aufgrund ihres niedrigen pHs nicht einfach zu realisieren. Auch die erfindungsgemäß verwendeten milden Tenside sind schwierig zu verdicken.

Bevorzugt werden Hydrogelbildner als verdickende Substanz für die erfindungsgemäßen Reinigungszusammensetzungen verwendet.
Bevorzugte erfindungsgemäße Reinigungszusammensetzungen sind dadurch gekennzeichnet, dass mindestens eine verdickende Substanz auf Basis eines Polysaccharids und/oder auf Basis eines Homo- oder Copolymers, enthaltend das Monomer 2-Methyl-2[(1-oxo-2-propenyl)amino]-1-propan-sulfonsäure, die teilweise oder vollständig neutralisiert ist, enthalten ist.

Bevorzugte verdickende Substanzen auf Basis eines Polysaccharids sind ausgewählt aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin ausgewählt aus Xanthan-Gum, Dehydroxanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin ausgewählt aus Pectinen, Leinsamen-Gums, Agar-Agar, Carragheen (Carrageenan), Traganth, Gummi arabicum, Karayagummi, Taragummi, Gellan, Propylenglycolalginat, Alginsäuren und deren Salzen (Alginaten), insbesondere Natriumalginaten, Kaliumalginaten und Calciumalginaten, weiterhin - wenn auch weniger bevorzugt - ausgewählt aus physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen. Außerordentlich bevorzugt ist Dehydroxanthan-Gum.
Besonders bevorzugte erfindungsgemäße Reinigungszusammensetzung sind dadurch gekennzeichnet, dass mindestens eine verdickende Substanz auf Basis eines Polysaccharids, ausgewählt aus Xanthan-Gum, Dehydroxanthan-Gum, Carragheen, Guar-Gum, Alginaten, insbesondere Natriumalginaten, Gummi arabicum, Karaya-Gummi, Johannisbrotkernmehl, Leinsamen-Gums und Agar-Agar sowie Mischungen hiervon, enthalten ist.
Weitere besonders bevorzugte erfindungsgemäße Reinigungszusammensetzung sind dadurch gekennzeichnet, dass mindestens eine verdickende Substanz auf Basis eines Polysaccharids in einer Gesamtmenge von 0,2 - 2 Gew.-%, bevorzugt 0,5 - 1,5 Gew.-%, besonders bevorzugt 0,7 -1,2 Gew.-% und außerordentlich bevorzugt 0,8 - 1 Gew.-%, jeweils bezogen auf das Gewicht der gesamten Zusammensetzung, enthalten ist. Ebenfalls bevorzugt ist ein Gehalt von 0,5 - 1,5 Gew.-%, besonders bevorzugt 0,7 - 1,2 Gew.-% und außerordentlich bevorzugt 0,8 - 1 Gew.-%, jeweils bezogen auf das Gewicht der gesamten Zusammensetzung, an Dehydroxanthan-Gum. Dehydroxanthan-Gum ergab gegenüber den übrigen Polysaccharid-basierten Hydrogelbildnern im Zusammenwirken mit dem milde, aber schwierig zu verdickenden Tensidsystem, anwendungstechnisch besonders zufrieden stellende und lagerstabile Viskositäten.

Bevorzugte verdickende Substanzen auf Basis eines Homo- oder Copolymers, enthaltend das Monomer 2-Methyl-2[(1-oxo-2-propenyl)amino]-1-propansulfonsäure (AMPS), die teilweise oder vollständig neutralisiert ist, sind ausgewählt aus Poly-2-methyl-2[(1-oxo-2-propenyl)amino]-1-propansulfonsäure, Copolymeren von AMPS mit Acrylsäure, Copolymeren von AMPS mit Methacrylsäure, Copolymeren von AMPS mit Itaconsäure, Copolymeren von AMPS mit Maleinsäure, Copolymeren von AMPS mit 2-Hydroxyethylacrylat, Copolymeren von AMPS mit 2,3-Dihydroxypropylacrylat, Copolymeren von AMPS mit 2-Hydroxyethylmethacrylat, Copolymeren von AMPS mit 2,3-Dihydroxypropylmethacrylat, den ethoxylierten Derivaten der vorstehend genannten Ester mit einem Molekulargewicht zwischen 400 und 1000 g/mol, Copolymeren von AMPS mit Acrylamid, Copolymeren von AMPS mit Dimethylacrylamid sowie Copolymeren von AMPS mit Polyvinylpyrrolidon.

Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz.

Erfindungsgemäß besonders bevorzugte verdickende AMPS-haltige Polymere sind teilweise oder vollständig neutralisierte, vernetzte Copolymere aus 2-Methyl-2[(1-oxo-2-propenyl)amino]-1-propan-sulfonsäure (AMPS) und Vinylpyrrolidon. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Vinylpyrrolidon, die in Form des Ammoniumsalzes kommerziell erhältlich sind, z. B. unter dem Handelsnamen Aristoflex^{®} AVC von der Firma Clariant.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Hydroxyethylacrylat. Erfindungsgemäß außerordentlich bevorzugt sind teilweise oder vollständig neutralisierte, vernetzte Copolymere aus 2-Methyl-2[(1-oxo-2-propenyl)amino]-1-propansulfon-säure (AMPS) und Hydroxyethylacrylat sind z. B. unter dem Handelsnamen Simulgel^{®}NS von der Firma Seppic kommerziell erhältlich.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylsäure, wobei die Säuregruppen teilweise oder vollständig neutralisiert sein können. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylsäure sind z. B. unter den Handelsnamen Simulgel^{®} EG, Sepiplus^{®} 400 und Simulgel^{®} EPG von Seppic kommerziell erhältlich.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Dimethylacrylamid. Erfindungsgemäß außerordentlich bevorzugt sind teilweise oder vollständig neutralisierte, vernetzte Copolymere aus 2-Methyl-2[(1-oxo-2-propenyl)amino]-1-propan-sulfonsäure (AMPS) und Dimethylacrylamid sind z. B. unter dem Handelsnamen Simulgel^{®} SMS 88 von Seppic kommerziell erhältlich.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylamid. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylamid sind z. B. unter den Handelsnamen Simulgel^{®} 600, Sepiplus^{®} 400 und Sepigel^{®} 305 von Seppic kommerziell erhältlich.

Sofern es sich bei den erfindungsgemäß besonders bevorzugten verdickenden Polymeren um inverse Polymerlatices handelt, ist diesen gemein, dass sie mindestens ein Öl sowie mindestens einen Öl-in-Wasser-Emulgator enthalten. Besonders bevorzugte Öle sind ausgewählt aus weißen Mineralölen, Squalan, Polyisobuten und hydriertem Polyisobuten. Besonders bevorzugte Öl-in-Wasser-Emulgatoren sind ausgewählt aus den Ethylenoxid-Addukten von Verbindungen wie bevorzugt Sorbitanoleat, Ricinusöl, das gewünschtenfalls gehärtet ist, Sorbitanlaurat und Laurylalkohol, wobei der Ethoxylierungsgrad so hoch ist, dass die Emulgatoren als Öl-in-Wasser-Emulgator wirken. Weitere besonders bevorzugte Öl-in-Wasser-Emulgatoren sind ausgewählt aus Alkyl-(oligo)-glucosiden, insbesondere Alkyl-(oligo)-glucosiden mit einem linearen C₆-, C₈-, C₁₀- und C₁₂-Alkylrest sowie Mischungen hiervon, insbesondere C₈/C₁₀-Alkyloligoglucoside, besonders bevorzugt C₈/C₁₀-Alkyloligoglucoside, jeweils mit einem Oligomerisierungsgrad von 1,1 - 1,4.

Sofern es sich bei den erfindungsgemäß besonders bevorzugten verdickenden Polymeren um inverse Polymerlatices handelt, ist diesen gemein, dass sie bevorzugt einen Polymergehalt von 30 - 90 Gew.-%, besonders bevorzugt 50 - 80 Gew.-% und außerordentlich bevorzugt 60 - 75 Gew.-%, jeweils bezogen auf den gesamten Latex, aufweisen.
Bezogen auf die gesamte erfindungsgemäße kosmetische Zusammensetzung, ist das verdickende Copolymer mit mindestens einem Monomer mit starker Säurefunktion und mindestens einem neutralen Monomer oder Monomer mit schwacher Säurefunktion bevorzugt in Gesamtmengen von 0,1 - 5 Gew.-%, besonders bevorzugt 0,3 - 3 Gew.-% und außerordentlich bevorzugt 0,5 - 2,5 Gew.-%, jeweils bezogen auf die gesamte erfindungsgemäße Zusammensetzung, enthalten.

Weitere besonders bevorzugte erfindungsgemäße Reinigungszusammensetzung sind dadurch gekennzeichnet, dass mindestens eine verdickende Substanz auf Basis eines AMPS-haltigen Homo-oder Copolymers in einer Gesamtmenge von 0,2 - 2 Gew.-%, bevorzugt 0,3 - 1,5 Gew.-%, besonders bevorzugt 0,4 - 1,0 Gew.-% und außerordentlich bevorzugt 0,5 - 0,8 Gew.-%, jeweils bezogen auf das Gewicht an Polymeraktivsubstanz in der gesamten erfindungsgemäßen Zusammensetzung, enthalten ist.

In einer weiteren bevorzugten Ausführungsform sind die erfindungsgemäßen Reinigungszusammensetzungen dadurch gekennzeichnet, dass sie als beschichtetes Substrat, insbesondere als Patch, Pflaster oder Gelpatch, konfektioniert sind. Hierzu enthält die erfindungsgemäße Zusammensetzung mindestens eine verdickende Substanz auf Basis eines Polysaccharids und/oder auf Basis eines Homo- oder Copolymers, enthaltend das Monomer 2-Methyl-2[(1-oxo-2-propenyl)amino]-1-propan-sulfonsäure, die teilweise oder vollständig neutralisiert ist, in einer Gesamtmenge von 2 - 20 Gew.-%, bevorzugt 3 - 15 Gew.-%, besonders bevorzugt 4 - 10 Gew.-% und außerordentlich bevorzugt 5 - 8 Gew.-%, jeweils bezogen auf das Gewicht an Polymeraktivsubstanz in der gesamten erfindungsgemäßen Zusammensetzung.

In einer weiteren bevorzugten Ausführungsform sind die erfindungsgemäßen Reinigungszusammensetzungen dadurch gekennzeichnet, dass sie als getränktes Substrat, insbesondere als Tuch, Wipe, Nonwoven, Pad oder Schwamm, konfektioniert sind.

In einer weiteren bevorzugten Ausführungsform enthalten die erfindungsgemäßen Zusammensetzungen zusätzlich zu dem obligatorischen 1,6-Hexandiol mindestens ein weiteres wasserlösliches Polyol, ausgewählt aus wasserlöslichen Diolen (zweiwertigen Alkoholen), Triolen (zweiwertigen Alkoholen) und höherwertigen Alkoholen sowie Polyethylenglycolen. Derartige Wirkstoffe können die Stabilitätseigenschaften der erfindungsgemäßen Zusammensetzungen in überraschender Weise positiv beeinflussen.
Erfindungsgemäß wird unter Wasserlöslichkeit eine Löslichkeit von wenigstens 5 Gew.-% bei 20 °C verstanden. 5 Gew.-% bedeutet, dass 5 g des Polyols in 95 g Wasser bei 20 °C löslich sind.
Unter den Diolen eignen sich bevorzugt C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, 1,2-Hexandiol, 1,2-Octandiol sowie 1,2-Decandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.
Bevorzugte erfindungsgemäße Zusammensetzungen enthalten zusätzlich zu 1,6-Hexandiol mindestens ein wasserlösliches Polyol bevorzugt in Gesamtmengen von (ohne 1,6-Hexandiol) 1 - 50 Gew.-%, besonders bevorzugt 4 - 15 Gew.-% und außerordentlich bevorzugt 6 - 10 Gew.-%, jeweils bezogen auf die gesamte Zusammensetzung.

Weitere bevorzugte erfindungsgemäße Zusammensetzungen sind dadurch gekennzeichnet, dass mindestens ein alkyl- oder hydroxyalkylsubstituiertes Harnstoffderivat enthalten ist. Bevorzugte alkyl-oder hydroxyalkylsubstituierte Harnstoffderivate sind solche der allgemeinen Formel (UREA) 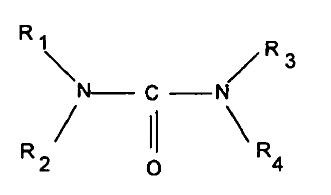 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 - 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.
Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (UREA) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind dadurch gekennzeichnet, dass der mindestens eine alkyl- oder hydroxyalkylsubstituierte Harnstoff der allgemeinen Formel (UREA) ausgewählt ist aus Bis-N,N'-(2-hydroxyethyl)harnstoff.
Erfindungsgemäß besonders bevorzugte Zusammensetzungen sind dadurch gekennzeichnet, dass sie mindestens einen alkyl- oder hydroxyalkylsubstituierten Harnstoff gemäß Formel (UREA), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance von National Starch als ca. 45 - 55 Gew.-%ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens einen kosmetischen Wirkstoff, der die Eigenschaften, insbesondere die pflegenden Eigenschaften der erfindungsgemäßen Zusammensetzungen in überraschender Weise positiv beeinflussen kann, ausgewählt aus:
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen,
d) Vitaminen, Provitaminen und Vitaminvorstufen der Gruppen A, B, C, E und H und den Estern der vorgenannten Substanzen,
e) Flavonoiden und Flavonoid-reichen Pflanzenextrakten,
f) Isoflavonoiden und Isoflavonoid-reichen Pflanzenextrakten,
g) alkyl- oder hydroxyalkylsubstituierten Harnstoffderivaten,
h) Ubichinon und Ubichinol sowie deren Derivaten,
i) Silymarin,
j) natürlich vorkommenden Xanthin-Derivaten, ausgewählt aus Coffein, Theophyllin, Theobromin und Aminophyllin,
k) Ectoin,
l) Kreatin,
m) Olivenblattextrakten, Ursolsäure, Oleanol und/oder Oleanolsäure,
n) Mono- und Polyhydroxystilbenen, insbesondere Resveratrol, und deren Estern,
o) Derivaten von methyliertem Silanol,
p) Phytinsäure,
q) Extrakten aus Maiskörnern (Zea Mays (Corn) Kernel Extract),
r) Extrakten aus Haferkörnern (Avena Sativa (Oat) Kernel Extract),
s) Saccharomyces/Xylinum/Black Tea Ferment,
t) Apfelkernextrakten (Pyrus Malus (Apple) Fruit Extract),
u) Lotuskeim-Extrakten (Nelumbo Nucifera Germ Extract),
v) Rotweinextrakten,
w) Traubenkernextrakten (Vitis Vinifera (Grape) Seed Extract), die bevorzugt aus der Chardonnay-Traube stammen,
x) Extrakten aus Schwarzen Holunderblüten (Sambucus Nigra Flower Extract),
y) Wirkstoffen, die die beta-Endorphinsynthese in Keratinozyten stimulieren,
z) hautaufhellenden Wirkstoffen,
aa) Wirkstoffen, die die Prostaglandinsynthese und/oder die Leukotrien-Synthese inhibieren, sowie
bb) sebumregulierenden Wirkstoffen.

Ein weiterer Gegenstand der vorliegenden Anmeldung ist die nicht-therapeutische, kosmetische Verwendung einer Reinigungszusammensetzung gemäß einem der Patentansprüche 1 - 13 zur Hautaufhellung, zur Aufhellung von Altersflecken, zur Egalisierung der Gesichtsfarbe, zur Verringerung der Tiefe von Hautfalten und/oder zur Egalisierung des Hautreliefs.

Ein weiterer Gegenstand der vorliegenden Anmeldung ist ein nicht-therapeutisches, kosmetisches Verfahren zur Hautaufhellung, zur Aufhellung von Altersflecken, zur Egalisierung der Gesichtsfarbe, zur Verringerung der Tiefe von Hautfalten und/oder zur Egalisierung des Hautreliefs, bei dem eine Reinigungszusammensetzung gemäß einem der Ansprüche 1 - 13 auf die Haut aufgetragen und nach einer Einwirkzeit von 1 Sekunde, bevorzugt 10 Sekunden, bis 5 Minuten wieder entfernt, bevorzugt abgespült und/oder abgewischt, wird.

### Ausführungsbeispiele

Die folgenden Beispiele sollen die Erfindung verdeutlichen, ohne sie hierauf zu beschränken.

### Anti-Falten-Reinigungsgel

| Bestandteil | Menge [Gew.-%] |
|---|---|
| 1,6-Hexandiol | 6,0 |
| Glycolsäure | 3,8 |
| Decylglucosid | 3,0 |
| Natriumhydroxid | 1,05 |
| Dehydroxanthan Gum | 1,03 |
| 2-Amino-5-guanidinopentansäure | 0,4 |
| PEG-7 Glyceryl Cocoate | 0,4 |
| Parfüm | 0,1 |
| Olivenblattextrakt | 0,2 |
| Wasser | ad 100 |

Die Zusammensetzung hat einen pH-Wert von 4 und eine Viskosität von 15.000 - 19.000 mPas (bei 20°C, Haake Viskosimeter, Spindel TC, 4 UPM).

### Anti-Altersflecken-Reinigungsgel

| Bestandteil | Menge [Gew.-%] |
|---|---|
| 1,6-Hexandiol | 8,0 |
| Glycolsäure | 4,0 |
| Decylglucosid | 2,5 |
| DIPALMITOYLETHYL HYDROXYETHYLMONIUM METHOSULFATE | 0,5 |
| Natriumhydroxid | 1,5 |
| Dehydroxanthan Gum | 1,03 |
| 1,3-Butylenglycol | 7,0 |
| PEG-7 Glyceryl Cocoate | 0,4 |
| Parfüm | 0,1 |
| Taurin | 0,2 |
| Resveratrol | 0,05 |
| Wasser | ad 100 |

### Complexion egalizer

| Bestandteil | Menge [Gew.-%] |
|---|---|
| 1,6-Hexandiol | 6,0 |
| Glycolsäure | 3,6 |
| Decylglucosid | 2,5 |
| DIPALMITOYLETHYL HYDROXYETHYLMONIUM METHOSULFATE | 0,5 |
| Natriumhydroxid | 1,1 |
| 2-Amino-5-guanidinopentansäure | 0,5 |
| Simulgel NS | 1,5 |
| 1,3-Butylenglycol | 7,0 |
| PEG-7 Glyceryl Cocoate | 0,4 |
| Parfüm | 0,1 |
| N,N'-Bis(2-hydroxyethyl)harnstoff | 2,0 |
| Harnstoff | 0,2 |
| Resveratrol | 0,05 |
| Ammoniumlactat | 0,1 |
| Wasser | ad 100 |

Die vorstehend aufgeführten erfindungsgemäßen Beispielzusammensetzungen wurden mit der Hand auf die Gesichtshaut aufgetragen und nach 1, 2, 3, 4, 5, 6, 7 oder 8 Minuten Einwirkzeit mit Wasser abgespült.
Im Vergleich zwischen den erfindungsgemäßen Zusammensetzungen und Zusammensetzungen ohne 1,6-Hexandiol, aber mit Benzoesäure, Parabenen oder Phenoxyethanol in üblichen Mengen, gaben die Probanden an, nach der Anwendung einer erfindungsgemäßen Zusammensetzung deutlich weniger oder sogar keine Hautreizungen zu verspüren als nach der Anwendung einer Vergleichszusammensetzung.
Die erfindungsgemäß behandelte Haut der Probanden zeigte nach 4 Wochen regelmäßiger Anwendung (2 - 3mal pro Woche) einen deutlich gleichmäßigeren Teint.

## Patentansprüche

1. Reinigungszusammensetzung für die Haut und/oder die Haare, enthaltend
a) Wasser,
b) ein mildes Tensidsystem, umfassend 0,5 - 20 Gew.-% Gesamttensidgehalt,
c) mindestens 2 Gew.-% mindestens einer alpha-Hydroxycarbonsäure, alpha-Ketocarbonsäure und/oder beta-Hydroxycarbonsäure,
d) mindestens 2 Gew.-% 1,6-Hexandiol,
wobei alle Mengenangaben auf das Gewicht der gesamten Zusammensetzung bezogen sind und wobei die Zusammensetzung frei ist von
e) Cocamidopropylbetain,
f) den Aniontensiden Seifen, Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Fettsäureamid(ether)sulfaten, Sulfotriglyceriden, Amidseifen, und Alkyl(ether)phosphaten,
g) Benzoesäure, 4-Hydroxybenzoesäure, den Salzen und den Estern dieser Säuren, Phenoxyethanol, Phenoxypropanol, lodopropynyl Butylcarbamate und 1,3-Bis(hydroxymethyl)-5,5-dimethylimidazolidin-2,4-dion,
h) und wobei die gesamte Zusammensetzung einen pH-Wert im Bereich von 3,5 - 4,5 aufweist.

2. Reinigungszusammensetzung gemäß Anspruch 1, **dadurch gekennzeichnet, dass** 4 - 20 Gew.-%, bevorzugt 6 - 10 Gew.-% 1,6-Hexandiol enthalten sind.

3. Reinigungszusammensetzung gemäß einem der vorstehenden Ansprüche, **dadurch gekennzeichnet, dass** mindestens ein C₆-C₁₆-Alkyl- oder Alkenylglycosid mit einem Oligomerisierungsgrad von 1 - 3 enthalten ist.

4. Reinigungszusammensetzung gemäß einem der vorstehenden Ansprüche, **dadurch gekennzeichnet, dass** mindestens ein kationisches Tensid, ausgewählt aus der Tensidklasse der Esterquats, enthalten ist.

5. Reinigungszusammensetzung gemäß einem der vorstehenden Ansprüche, **dadurch gekennzeichnet, dass** nur Tenside mit einem HET - CAM Reizscore kleiner als 1,5 enthalten sind.

6. Reinigungszusammensetzung gemäß einem der vorstehenden Ansprüche, **dadurch gekennzeichnet, dass** der Gesamttensidgehalt maximal 4,5 Gew.-%, bezogen auf die gesamte Zusammensetzung, beträgt.

7. Reinigungszusammensetzung gemäß einem der vorstehenden Ansprüche, **dadurch gekennzeichnet, dass** die alpha-Hydroxycarbonsäure ausgewählt ist aus Glycolsäure.

8. Reinigungszusammensetzung gemäß einem der vorstehenden Ansprüche, **dadurch gekennzeichnet, dass** mindestens eine Guanidino-substituierte C₁ - C₁₀-Carbonsäure, insbesondere mindestens eine alpha-Amino-omega-Guanidino-C₂ - C₁₀-Carbonsäure enthalten ist.

9. Reinigungszusammensetzung gemäß einem der vorstehenden Ansprüche, **dadurch gekennzeichnet, dass** das Gewichtsverhältnis von Gesamtmenge an alpha-Hydroxycarbonsäure, alpha-Ketocarbonsäure und beta-Hydroxycarbonsäure zur Gesamtmenge an Guanidino-substituierten C₁ - C₁₀-Carbonsäuren 4 - 20, bevorzugt 6 - 10, beträgt.

10. Reinigungszusammensetzung gemäß einem der vorstehenden Ansprüche, **dadurch gekennzeichnet, dass** mindestens eine verdickende Substanz auf Basis eines Polysaccharids und/ oder auf Basis eines Homo- oder Copolymers, enthaltend das Monomer 2-Methyl-2[(1-oxo-2-propenyl)-amino]-1-propansulfonsäure, die teilweise oder vollständig neutralisiert ist, enthalten ist.

11. Reinigungszusammensetzung gemäß einem der vorstehenden Ansprüche, **dadurch gekennzeichnet, dass** mindestens eine verdickende Substanz auf Basis eines Polysaccharids, ausgewählt aus Xanthan-Gum, Dehydroxanthan-Gum, Carragheenan, Guar-Gum, Alginaten, insbesondere Natriumalginaten, Gummi arabicum, Karaya-Gummi, Johannisbrotkernmehl, Leinsamen-Gums und Agar-Agar sowie Mischungen hiervon, enthalten ist.

12. Reinigungszusammensetzung gemäß einem der vorstehenden Ansprüche, **dadurch gekennzeichnet, dass** sie als getränktes Substrat, insbesondere als Tuch, Wipe, Nonwoven, Pad oder Schwamm, konfektioniert ist.

13. Reinigungszusammensetzung gemäß einem der vorstehenden Ansprüche, **dadurch gekennzeichnet, dass** sie als beschichtetes Substrat, insbesondere als Patch, Pflaster oder Gelpatch, konfektioniert ist.

14. Nicht-therapeutische, kosmetische Verwendung einer Reinigungszusammensetzung gemäß einem der Ansprüche 1 - 13 zur Hautaufhellung, zur Aufhellung von Altersflecken, zur Egalisierung der Gesichtsfarbe, zur Verringerung der Tiefe von Hautfalten und/oder zur Egalisierung des Hautreliefs.

15. Nicht-therapeutisches, kosmetisches Verfahren zur Hautaufhellung, zur Aufhellung von Altersflecken, zur Egalisierung der Gesichtsfarbe, zur Verringerung der Tiefe von Hautfalten und/oder zur Egalisierung des Hautreliefs, **dadurch gekennzeichnet, dass** eine Reinigungszusammensetzung gemäß einem der Ansprüche 1 - 13 auf die Haut aufgetragen und nach einer Einwirkzeit von 1 Sekunde, bevorzugt 10 Sekunden, bis 5 Minuten wieder entfernt, bevorzugt abgespült und/oder abgewischt, wird.

## Claims

1. A cleaning composition for the skin and/or the hair containing
a) water
b) a gentle surfactant system comprising 0.5-20 wt.% total surfactant content,
c) at least 2 wt.% of at least one alpha-hydroxycarboxylic acid, alpha-ketocarboxylic acid and/or beta-hydroxycarboxylic acid,
d) at least 2 wt.% 1,6-hexanediol,
wherein all the stated quantities relate to the weight of the entire composition and wherein the composition is free of
e) cocamidopropyl betaine,
f) anionic surfactant soaps, alkylbenzene sulfonates, alkane sulfonates, olefin sulfonates, alkyl ether sulfonates, glyceryl ether sulfonates, α-methyl ester sulfonates, sulfofatty acids, alkyl sulfates, fatty alcohol ether sulfates, fatty acid amide (ether) sulfates, sulfotriglycerides, amide soaps and alkyl (ether) phosphates,
g) benzoic acid, 4-hydroxybenzoic acid, the salts and esters of these acids, phenoxyethanol, phenoxypropanol, iodopropynyl butylcarbamate and 1,3-bis(hydroxymethyl)-5,5-dimethylimidazolidine-2,4-dione,
h) and wherein the entire composition exhibits a pH in the range from 3.5-4.5.

2. A cleaning composition according to claim 1, **characterised in that** 4-20 wt.%, preferably 6-10 wt.% of 1,6-hexanediol are present.

3. A cleaning composition according to any one of the preceding claims, **characterised in that** at least one C₆-C₁₆ alkyl or alkenyl glycoside with a degree of oligomerisation of 1-3 is present.

4. A cleaning composition according to any one of the preceding claims, **characterised in that** at least one cationic surfactant selected from the class of ester quat surfactants is present.

5. A cleaning composition according to any one of the preceding claims, **characterised in that** only surfactants with a HET-CAM irritation score of less than 1.5 are present.

6. A cleaning composition according to any one of the preceding claims, **characterised in that** the total surfactant content amounts to at most 4.5 wt.% relative to the entire composition.

7. A cleaning composition according to any one of the preceding claims, **characterised in that** the alpha-hydroxycarboxylic acid is selected from glycolic acid.

8. A cleaning composition according to any one of the preceding claims, **characterised in that** at least one guanidino-substituted C₁-C₁₀ carboxylic acid, in particular at least one alpha-amino-omega-guanidino-C₂-C₁₀-carboxylic acid is present.

9. A cleaning composition according to any one of the preceding claims, **characterised in that** the weight ratio of the total quantity of alpha-hydroxycarboxylic acid, alpha-ketocarboxylic acid and beta-hydroxycarboxylic acid to the total quantity of guanidino-substituted C₁-C₁₀ carboxylic acids amounts to 4-20, preferably 6-10.

10. A cleaning composition according to any one of the preceding claims, **characterised in that** at least one thickening substance based on a polysaccharide and/or based on a homo- or copolymer containing the monomer 2-methyl-2[(1-oxo-2-propenyl)-amino]-1-propanesulfonic acid, which is partially or completely neutralised, is present.

11. A cleaning composition according to any one of the preceding claims, **characterised in that** at least one thickening substance based on a polysaccharide selected from xanthan gum, dehydroxanthan gum, carrageenan, guar gum, alginates, in particular sodium alginates, gum arabic, karaya gum, locust bean flour, linseed gums and agar-agar and mixtures thereof is present.

12. A cleaning composition according to any one of the preceding claims, **characterised in that** it is formulated as an impregnated substrate, in particular as a cloth, wipe, nonwoven, pad or sponge.

13. A cleaning composition according to any one of the preceding claims, **characterised in that** it is formulated as a coated substrate, in particular as a patch, dressing or gel patch.

14. Non-therapeutic, cosmetic use of a cleaning composition according to any one of claims 1-13 for skin lightening, for lightening age spots, for equalising facial colour, for reducing the depth of skin folds and/or for equalising skin relief.

15. A non-therapeutic, cosmetic method for skin lightening, for lightening age spots, for equalising facial colour, for reducing the depth of skin folds and/or for equalising skin relief, **characterised in that** a cleaning composition according to any one of claims 1-13 is applied onto the skin and, after an exposure time of 1 second, preferably of 10 seconds to 5 minutes, is removed again, preferably rinsed and/or wiped off.

## Revendications

1. Composition de nettoyage pour la peau et/ou pour les cheveux, contenant :
a) de l'eau ;
b) un système tensioactif doux possédant une teneur totale en agents tensioactifs de 0,5 à 20 % en poids ;
c) au moins 2 % en poids d'au moins un acide alpha-hydroxycarboxylique, un acide alpha-cétocarboxylique et/ou un acide bêta-hydroxycarboxylique ;
d) au moins 2 % en poids du 1,6-hexanediol ;
toutes les indications quantitatives se rapportant au poids de la composition globale et la composition étant exempte de :
e) cocamidopropylbétaïne ;
f) les savons tensioactifs anioniques, les alkylbenzènesulfonates, les alcanesulfonates, les oléfinesulfonates, les alkyléthersulfonates, les glycéroléthersulfonates, les sulfonates d'esters alpha-méthyliques, les acides sulfogras, les alkylsulfates, les éthersulfates d'alcools gras, les (éther)sulfates d'amides d'acides gras, les sulfotriglycérides, les savons amidiques et les alkyl(éther)phosphates ;
g) l'acide benzoïque, l'acide 4-hydroxybenzoïque, les sels et les esters de ces acides, le phénoxyéthanol, le phénoxypropanol, l'iodopropynyl butylcarbamate et la 1,3-bis(hydroxyméthyl)-5,5-diméthylimidazolidine-2,4-dione ;
h) et la composition totale présentant une valeur de pH dans la plage de 3,5 à 4,5.

2. Composition de nettoyage selon la revendication 1, **caractérisée en ce qu'**elle contient de 4 à 20 % en poids, de préférence de 6 à 10 % en poids du 1,6-hexanediol.

3. Composition de nettoyage selon l'une quelconque des revendications précédentes, **caractérisée en ce qu'**elle contient au moins un alkyl(en C₆-C₁₆)- ou alcényl-glycoside possédant un degré d'oligomérisation de 1 à 3.

4. Composition de nettoyage selon l'une quelconque des revendications précédentes, **caractérisée en ce qu'**elle contient au moins un agent tensioactif cationique, choisi parmi la classe des agents tensioactifs des esterquats.

5. Composition de nettoyage selon l'une quelconque des revendications précédentes, **caractérisée en ce qu'**elle contient uniquement des agents tensioactifs dont l'indice d'irritation HET - CAM est inférieur à 1,5.

6. Composition de nettoyage selon l'une quelconque des revendications précédentes, **caractérisée en ce que** la teneur totale en agents tensioactifs s'élève au maximum à 4,5 % en poids, rapportés à la composition totale.

7. Composition de nettoyage selon l'une quelconque des revendications précédentes, **caractérisée en ce que** l'acide alpha-hydroxycarboxylique est choisi parmi l'acide glycolique.

8. Composition de nettoyage selon l'une quelconque des revendications précédentes, **caractérisée en ce qu'**elle contient au moins un acide carboxylique en C₁-C₁₀ substitué par un groupe guanidino, en particulier au moins un acide alpha-amino-oméga-guanidinocarboxylique en C₂-C₁₀.

9. Composition de nettoyage selon l'une quelconque des revendications précédentes, **caractérisée en ce que** le rapport pondéral de la quantité totale de l'acide alpha-hydroxycarboxylique, de l'acide alpha-cétocarboxylique et d'acide bêta-hydroxycarboxylique par rapport à la quantité totale d'acides carboxyliques en C₁-C₁₀ substitués par un groupe guanidino s'élève à 4-20, de préférence à 6-10.

10. Composition de nettoyage selon l'une quelconque des revendications précédentes, **caractérisée en ce qu'**elle contient au moins une substance épaississante à base d'un polysaccharide et/ou à base d'un homopolymère ou d'un copolymère contenant à titre de monomère, l'acide 2-méthyl-2[(1-oxo-2-propényl)-amino]-1-propansulfonique qui est neutralisé en partie ou complètement.

11. Composition de nettoyage selon l'une quelconque des revendications précédentes, **caractérisée en ce qu'**elle contient au moins une substance épaississante à base d'un polysaccharide, choisi parmi la gomme de xanthane, la gomme de déshydroxyxanthane, le carraghénane, la gomme de guar, des alginates en particulier des alginates de sodium, la gomme arabique, la gomme de karaya, la farine de graine de caroube, la gomme de graine de lin et l'agar-agar, ainsi que leurs mélanges.

12. Composition de nettoyage selon l'une quelconque des revendications précédentes, **caractérisée en ce qu'**elle est confectionnée sous la forme d'un substrat imprégné, en particulier sous la forme d'une lingette, d'un tissu, d'un non-tissé, d'un tampon ou d'une éponge.

13. Composition de nettoyage selon l'une quelconque des revendications précédentes, **caractérisée en ce qu'**elle est confectionnée sous la forme d'un substrat enduit, en particulier sous la forme d'un patch, d'un emplâtre ou d'un « gelpatch ».

14. Utilisation cosmétique non thérapeutique d'une composition de nettoyage selon l'une quelconque des revendications 1 à 13, pour l'éclaircissement de la peau, pour l'éclaircissement de taches liées à l'âge, pour l'égalisation de la couleur du visage, pour la réduction des profondeurs des plis de la peau et/ou pour l'égalisation du relief cutané.

15. Procédé cosmétique non thérapeutique pour l'éclaircissement de la peau, pour l'éclaircissement de taches liées à l'âge, pour l'égalisation de la couleur du visage, pour la réduction des profondeurs des plis de la peau et/ou pour l'égalisation du relief cutané, **caractérisé en ce qu'**on applique sur la peau une composition de nettoyage selon l'une quelconque des revendications 1 à 13, et après l'avoir laissé agir pendant un laps de temps de 1 seconde, de préférence de 10 secondes jusqu'à 5 minutes, on l'enlève à nouveau, de préférence par rinçage et/ou par essuyage.
